(19) Europäisches Patentamt
European Patent Office
Office européen des brevets

(11) Publication number: **0 175 581**
**B1**

(12) **EUROPEAN PATENT SPECIFICATION**

(45) Date of publication of patent specification: 22.08.90

(51) Int. Cl.[5]: C 07 C 231/08, C 07 D 213/81

(21) Application number: 85306670.2

(22) Date of filing: 19.09.85

(54) **Hydration process of nitrile compound.**

(30) Priority: 20.09.84 JP 195721/84

(43) Date of publication of application: 26.03.86 Bulletin 86/13

(45) Publication of the grant of the patent: 22.08.90 Bulletin 90/34

(84) Designated Contracting States: AT DE FR GB IT NL

(56) References cited:
US-A-3 944 609

CHEMICAL ABSTRACTS, vol. 84, no. 4, 26th January 1976, page 23, abstract no. 18027h, Columbus, Ohio, US; & JP-A-75 93 919

CHEMICAL ABSTRACTS, vol. 101, no. 8, 20th August 1984, page 1, abstract no. 55548u, Columbus, Ohio, US; I.O. MIKHAILISHIN et al.: "Kinetics of the hydration of acrylonitrile on iron-copper catalysts"

(73) Proprietor: MITSUI TOATSU CHEMICALS, Inc.
2-5 Kasumigaseki 3-chome
Chiyoda-Ku Tokyo 100 (JP)

(72) Inventor: Asano, Shiro
325, Ayai Shataku, 4-7, Kamo
Takaishi-shi Osaka-fu (JP)
Inventor: Kitagawa, Jun
4-6-20, Hikoshimasakomachi
Shimonoseki-shi Yamaguchi-ken (JP)

(74) Representative: Stuart, Ian Alexander et al
MEWBURN ELLIS & CO. 2/3 Cursitor Street
London EC4A 1BQ (GB)

EP 0 175 581 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for hydrating a nitrile compound in the presence of a Raney copper catalyst in a liquid-phase suspended-bed to synthesize its corresponding acid amide compound, and specifically to an improved process for the hydration of a nitrile compound. It preferably uses a catalyst which retains high activity; and is advantageous where the catalyst is separated by sedimentation and/or filtration to recover the liquid reaction mixture.

Regarding the hydration of nitrile compounds in liquid phases and in the presence of Raney copper catalysts for the synthetic preparation of their corresponding acid amines, many proposals have heretofore been made including, for example, the preparation of nicotinamide (Japanese Patent Publication No. 22710/1973), the synthesis of acrylamide (US Patent No. 4056565), the effectiveness of addition of a copper salt selected from copper sulfate, copper nitrate, copper halides and the copper salts of fatty acids in an amount of 5—300 ppm based on water to a reaction system (US Patent Nos. 3911009 and 396233, the improved development of Raney copper alloys (Japanese Patent Publication No. 26910/1980), and the preparation of copper catalysts inclusive of Raney copper catalysts (U.S. Patent No. 3,767,706).

On the other hand, the following illustrative proposals have also been known on the effects of iron component in catalyst or reaction systems useful for the hydration reactions of nitrile compounds. Namely, Japanese Patent Laid-Open No. 13312/1975 discloses that the service life of the catalyst can be prolonged by causing one or more of various nitrates inclusive of iron nitrate to exist in an amount of 1—600 ppm as nitrate radicals in a system useful for the hydration reaction of a nitrile compound to be effected in the presence of a metallic copper-base catalyst such as Raney copper. Japanese Patent Laid-Open No. 115412/1976 discloses that metallic copper, which has been obtained by reducing a cuprous salt in the presence of a ferrous salt, is effective as a hydration catalyst for acrylonitrile and methacrylonitrile. Further, it is also proposed in Japanese Patent Publication No. 33612/1977 that a multi-component catalyst formed by incorporating iron and other metal components in Raney copper has higher activity than Raney copper itself. The last-mentioned Japanese patent publication contains an Example in which Raney copper having an iron content of about 3% was employed.

The present inventors have unexpectedly found that the following problems are also involved in reaction systems containing the above-described Raney copper catalyst with or without the above-mentioned compounds including iron added thereto, and catalyst systems composed of Raney copper and iron and other metal components added thereto.

First of all, the activity of each Raney copper catalyst varies considerably depending on its supply source. Even those prepared in accordance with the same preparation process, their activities are different from one preparation lot to another. When a powdery or granular Raney copper catalyst is used in a state suspended in a liquid reaction mixture, its readiness in sedimentation or filtration differs depending on its supply source or even its preparation lot upon separation of the catalyst from the liquid reaction mixture by sedimentation or filtration.

Needless to say, it is important to solve the problem that the activities of catalysts vary depending on their supply sources as mentioned above. When such a catalyst was poor in the sedimentation and filtration readiness in industrial production, unduly large sedimentation and filtration facilities were required. Furthermore, an economical loss such as unavoidable loss of the catalyst to an untolerably large amount due to its runoff and suspension of the operation due to clogging of filters or the like was also brought about. Since the readiness in sedimentation and filtration varied from one preparation lot of catalyst to another, no stable operation was feasible.

Under the aforementioned circumstances, it has been desired to impart high reactivity and good sedimentation and filtration readiness at the same levels to Raney copper catalysts.

The invention may enable one to provide a Raney copper catalyst capable of retaining high activity for a long service time when causing a nitrile compound to react with water in the presence of a Raney copper catalyst to prepare its corresponding acid amide.

The invention may enable one to provide a catalyst system featuring excellent sedimentation and filtration readiness upon effecting a reaction in the presence of a Raney copper catalyst in a suspended state and after the reaction, separating the catalyst and liquid reaction mixture from each other by sedimentation and filtration.

According to the invention, the process uses a Raney copper catalyst having an iron content of 0.1 to 0.4 wt.% based on the copper component in a process in which a nitrile compound is hydrated in the presence of a Raney copper catalyst while controlling the weight ratio of the nitrile compound to water at 60:40—5:95, the reaction temperature at 70—200°C and the conversion to the corresponding acid amide at 10—98%. The process involves effecting the hydration reaction in the presence of a Raney copper catalyst in a suspended state and after the reaction, separating the catalyst and liquid reaction mixture from each other by sedimentation and/or filtration to recover the liquid reaction mixture. Embodiments may be particularly effective, for example, in separating the catalyst through a filter disposed within a reactor and drawing the liquid reaction mixture out of the reactor or in separating the catalyst and liquid reaction mixture from each other by means of a thickener disposed within the reactor and then separating a small amount of the catalyst, which is still left in a state suspended in the liquid reaction mixture, by filtration either inside or outside the reactor.

In the above process, the acid amine compound can be synthesized at a stable level of turnout by using a small amount of Raney copper catalyst. Moreover, the separation of the catalyst and liquid reaction mixture by sedimentation and filtration can also be effected stably with ease. Accordingly, the above process has very large advantages for industrial production.

The present invention will hereinafter be exemplified in detail.

Nitrile compounds useful as starting materials in the present invention may include, for example, monovalent aliphatic nitriles such as acetonitrile and propionitrile, multivalent aliphatic nitriles such as malondinitrile, succinonitrile and adiponitrile, unsaturated aliphatic nitriles such as acrylonitrile and methacrylonitrile, aromatic nitriles such as benzonitrile and nicotinonitrile, and so on.

Reactions in which these nitrile compounds are hydrated into their corresponding acid amide compounds are shown by the general formula (I). When acrylonitrile, methacrylonitrile and nicotinonitrile are subjected, by way of example, to hydration reactions respectively, acrylamide, methacrylamide and nicotinoamide are prepared as illustrated in their corresponding chemical equations (2), (3) and (4).

$$R-CN + H_2O \rightarrow R-CONH_2 \quad (1)$$

$$CH_2=CH-CN + H_2O \rightarrow CH_2=CH-CONH_2 \quad (2)$$

$$CH_2=\underset{CH_3}{\underset{|}{C}}-CN + H_2O \rightarrow CH_2=\underset{CH_3}{\underset{|}{C}}-CONH_2 \quad (3)$$

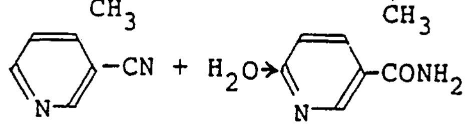

(4)

The Raney copper catalyst useful in the practice of the process of this invention is obtained by developing a so-called Raney copper alloy with an aqueous solution of a basic compound such as sodium hydroxide to leach out all soluble components from the alloy and then washing and removing the majority of the leached-out compounds and any excess basic compound with water or the like. Hence, its principal catalytic component is believed to be metallic copper. As soluble component or components in the alloy, may be mentioned aluminum and/or zinc. Of such soluble components, aluminum is suitable. The content of aluminum may preferably range from 30 to 70 wt.%. As the basic compound employed for the develoment of the alloy, it is possible to use inorganic compounds such as sodium hydroxide, sodium carbonate and potassium hydroxide and organic amine compounds such as trimethylamine with sodium hydroxide being preferred.

The catalyst must be in the form of either powder or small granules so that it may be used in a suspended-bed reactor. Catalysts having grain or particle sizes of 40 mesh or smaller as measured by the Tyler sieve system are generally used. In order to obtain these grain or particle sizes, it is necessary to develop powdery or granular Raney copper alloys having such grain or particle sizes or to develop Raney alloys of larger grain or particle sizes followed by their grinding into smaller grains or particles.

In order to enhance its activity or selectivity, the catalyst may additionally contain a second component such as nickel, molybdenum, silver or palladium. Where a nickel-containing Raney copper catalyst is desired by way of example, it may be prepared by developing a three-component Raney copper of aluminum-copper-nickel into a two-component Raney catalyst of copper-nickel for use in the above reaction. Here, no problem or inconvenience will be encountered even if the resulting two-component Raney catalyst has a higher content of the second component than that of copper. For example, the ratio of nickel as the second component to copper may be in the range between 55/45 and 85/15, and more typically it may be 70/30.

It is desired to keep the catalyst out of contact with oxygen or any oxygen-containing gas during its preparation and storage. Oxygen may not impair the activity of the catalyst or may inversely enhance its activity so long as the reaction of oxygen with the catalyst is controlled below a certain level. Beyond this level, its activity is however deleteriously affected and its sedimentation and filtration readiness from the liquid reaction mixture is hence impaired. If a catalyst, which has in advance been brought into contact with oxygen, is used for the hydration reaction of acrylonitrile, another problem will arise that the more byproducts such as ethylene cyanhydrin will be formed beside the intended acrylamide.

Synthesis of acrylamide through the hydration of acrylonitrile with such a Raney copper catalyst will next be illustrated by way of example.

A Raney copper catalyst is kept in a state suspended in a liquid reaction mixture and is used in either continuous or batchwise system.

The weight ratio of acrylonitrile to water to be subjected to the hydration reaction should be in the range 60:40—5:95, preferably 50:50—10:90. The preferable reaction temperature for the hydration reaction may be within the range of 70—150°C or more specifically 90—140°C. The conversion of acrylonitrile to acrylamide should be 10—98% and preferably 30—95%.

The above-described weight ratio of acrylonitrile to water, reaction temperature and conversion of acrylonitrile are applicable to general preparation conditions upon reacting nitrile compounds and water in the presence of a Raney copper catalyst in suspended state to prepare their corresponding acid amides. These conditions are particularly effective when a Raney copper catalyst having a very low iron content relative to the copper component is used.

If the iron content in a Raney copper catalyst to be used is too high, there will be brought about such inconvenience that as demonstrated by Examples to be given herein, the activity of the catalyst will be low to a significant extent, and the sedimentation and filtration readiness of the catalyst upon separation of the catalyst from the liquid reaction mixture will decrease rapidly with time during the reaction run and as specific inconvenience, the filtration resistance of a catalyst filter will increase. On the other hand, there is a practical limitation in minimizing the iron content in the catalyst. Therefore, the iron content may range from 0.1 to 0.4 wt.%.

Even under the above-described preparation conditions, the three components of the unreacted acrylonitrile, unreacted water and resulting acrylamide may not form any homogeneous liquid system in some instances. To avoid this problem, synthesized acrylamide may be added back again to the reaction system as a co-solvent or another inert co-solvent may also be used.

The interior of the reactor is kept at a pressure which is the sum of vapor pressures of the individual reaction components at the above-described temperature or the total of sum of such vapor pressures and the pressure of an inert gas such as nitrogen gas. The interior pressure may normally be within the range of from normal pressure to 20 atmospheres.

Oxygen dissolved in the catalyst slurry, acrylonitrile, water, co-solvent and the like, which are charged to the reactor, affects deleteriously on the activity of the catalyst, impairs the sedimentation and separation of the catalyst from the liquid reaction mixture and produces more byproducts such as ethylenecyanhydrin. It is therefore desirable to remove such accompanying oxygen fully. For the same reasons, it is desirable to maintain the interior of the reactor as an oxygen-free atmosphere.

The liquid reaction mixture obtained in the above reaction is then subjected to usual evaporation or distillation to obtain a concentrated aqueous solution of acrylamide. At the same time, substantially all the unreacted acrylonitrile and a portion of water are distilled off for their recovery. Although the thus-recovered acrylonitrile and water may be used in other application fields, they are generally used as the raw materials.

The hydration reaction of acrylonitrile has now been described in detail. The hydration reaction of methacrylonitrile is of the same notion and may hence be carried out in much the same way. Although the hydration reaction of nicotinonitrile is substantially of the same notion, a reaction temperature higher than that employed for acrylonitrile or methacrylonitrile, for example, a temperature up to 200°C may be employed.

The catalyst which has been used in the suspended state in the above-described hydration of the nitrile compound is usually separated immediately from the liquid reaction mixture. In some instances, it may however be separated from a concentrated liquid reaction mixture which is to be obtained subsequent to the above-described evaporation or distillation. The separation may generally be carried out by sedimentation and/or filtration. As a specific manner for effecting the sedimentation, it may be mentioned to use a thickener disposed within or outside the reactor or to use a centrifugal thickener. On the other hand, as a specific manner for effecting the filtration, may be mentioned to employ a filter disposed either inside or outside the reactor or to use a centrifugal filter. These sedimentation and filtration may also be combined.

The thus-separated catalyst may preferably be used repeatedly for the reactor. When a thickener is disposed within such a reactor in a continuous flow system as disclosed, for example, in U.S. Patent No. 3,985,806, the catalyst may be used continuously while holding the catalyst within the reactor as is.

When the reaction is effected in the above exemplified manner, the thus-separated liquid reaction mixture may be drawn out of the reactor and a small amount of fine particles of the catalyst, which is still left in a state suspended in the liquid reaction mixture, may be removed almost completely by filtration. It may also be feasible to dispose a filter within a reactor to remove the catalyst completely inclusive of its fines suspended in the liquid reaction mixture. These systems of thickener and filter, in combination or not, may be chosen as desired.

As mentioned above, the present invention makes use of a Raney copper catalyst having a particularly low iron content. As principal sources for the iron which is mixed in the Raney copper catalyst, may be contemplated (1) iron contained in aluminum, zinc and/or the like which are employed as soluble components in a Raney alloy, (2) iron mixed in as a result of wearing or corrosion of steel-made facilities employed upon grinding and classifying a Raney alloy to a desired range of particle sizes, (3) iron contained in sodium hydroxide and water which are both employed for the development of a Raney alloy, (4) iron mixed in as a result of wearing or corrosion of steel-made developing facilities.

In order to avoid the activity reduction of the catalyst and the reduction of its sedimentation and filtration readiness due to these various causes, it is necessary to implement various countermeasures so that the incorporation of iron by the above-mentioned causes can be minimized effectively.

As specific examples of the above-mentioned countermeasures, it may be mentioned (1) to use the limited brands or grades of aluminum or zinc to prepare the alloy, for example, to avoid use of aluminum or

zinc of such a high iron content as 0.4%; (2) to use grinding and classification facilities made of stainless steel instead of carbon steel; (3) to use sodium hydroxide having an iron content as low as possible and to avoid use of storage and transportation equipment made of carbon steel; (4) to use soft water, deionized water or the like as developing water and to avoid use of storage and transportation equipment made of carbon steel; (5) to use developing facilities made of stainless steel; etc.

By achieving the total control in such manners as mentioned above, it has become feasible for the first time to use a Raney catalyst having an iron content of 0.4 wt.% or less. Owing to the use of such a Raney catalyst, the above-described objects and effects have been fulfilled. Of the above-mentioned various countermeasures, the control of iron content in aluminum and the selection of material for the grinding and classification facilities are particularly important. Lack of these countermeasures appears to result in a great deal of iron left in a developed Raney copper.

The term "reduced sedimentation readiness of the catalyst" used in this invention as used herein means, for example, that when stirring is stopped in the course of use of an agitator-equipped reactor, the suspended particles or grains of the Raney copper catalyst become more difficult in sedimentation. When the reaction is effected for example in a reactor provided with a partition wall to form a catalyst sedimentation region (i.e., thickener), where the turburence is not applied to the catalyst slurry, and the precipitated catalyst is recycled to the catalyst-suspended region and the catalyst-separated liquid reaction mixture is drawn out of the reactor, the above term "reduced sedimentation readiness of the catalyst" used in this invention means that the thus-drawn liquid reaction mixture contains more catalyst which is still remaining in a suspended state without undergoing sedimentation. Increase of such suspended catalyst means a direct loss in the effective amount of the catalyst, leading to an increase of the catalyst consumption.

On the other hand, the term "reduced filtration readiness" as used herein means that the filtration resistance has been increased in a catalyst filter provided within or outside a reactor as will be demonstrated in Examples herein. Such a phenomenon will lead to a reduction of the production capacity.

This invention will next be described by the following Examples:

Example 1

A 1:1 alloy of copper and aluminum was ground and sifted to obtain Raney copper alloy powder having particle size less than 80 mesh. The alloy powder was developed with caustic soda in a usual manner, followed by its thorough washing with water to prepare a Raney copper catalyst. From a number of catalyst lots obtained in the above manner, those having iron contents of 0.10, 0.24, 0.41, 1.15 and 1.56%, based on their corresponding copper components, were used. The analysis of iron contents was effected by atomic absorption spectroscopy subsequent to dissolution of the catalysts in nitric acid.

Incidentally, the iron contents of the thus-chosen Raney alloys of 5 different lots before grinding and sifting and the material of a grinding mill employed are given in Table 1.

Using these five catalysts of different lots, the following batchwise reaction tests were conducted. Charged in a 100-ml flask equipped with a stirrer, thermometer and reflux condenser were 6.6 g of acrylonitrile, 36 g of water and 7 g of one of the catalysts. Since the catalyst was prepared and stored in a state immersed in water, about 7 g of water was accompanied when the catalyst was charged. Thus, this amount had in advance been deducted from the amount of water to be charged separately so that the total amount of water was set at 36 g.

The flask was heated externally over a bath to about 70°C. Thereafter, the contents were reacted for 2 hours while adjusting the temperature of the bath to maintain the internal temperature of the flask at 70—73°C.

Any dissolved oxygen had in advance been removed from the raw materials. In the subsequent operation, the penetration of air was avoided so as to prevent the catalyst from being brought into contact with oxygen. Two hours later, the flask was cooled to terminate the reaction and the liquid reaction mixture was analyzed by gas chromatography to determine the conversion of acrylonitrile to acrylamide. Results are summarized in Table 1.

TABLE 1

| Run No. | Iron content in alloy before grinding (wt.%; based on alloy) | Material of grinding mill | Iron content (%) based on copper | Conversion (%) | Classification |
|---|---|---|---|---|---|
| 1—1 | 0.04 | stainless steel | 0.10 | 67 | Example |
| 1—2 | 0.11 | ditto | 0.24 | 65 | ditto |
| 1—3 | 0.18 | ditto | 0.41 | 63 | ditto |
| 1—4 | 0.33 | ditto | 1.15 | 49 | Comp. Example |
| 1—5 | 0.57 | ditto | 1.56 | 40 | ditto |

Note: Stainless steel — SUS 304 was used.
Carbon steel — SS 41 was used.

When a Raney copper is used for the industrial production of acrylamide, its conversion is preferably 60% or higher in the above-described activity evaluation test. It can be seen from Table 1 that its embodiments of the invention can achieve such a preferable conversion.

Example 2

Using the two catalysts of Example 1 which had iron contents of 0.24, and 1.56% respectively, the following continuous flow reaction tests were conducted.

One hundred fifty parts of one of the catalysts were charged in a stainless steel reactor equipped with a stirrer and a built-in catalyst filter, to which acrylonitrile and water from both of which any dissolved oxygen had in advance been removed by blowing nitrogen gas thereinto were charged at flow rates of 300 and 700 parts by weight per hour respectively. The reaction was carried out at 120°C while stirring the reaction system to maintain the catalyst in a suspended state. As a reaction promoter, copper nitrate was added to the water to be fed in such an amount that its concentration reached 20 ppm of the liquid reaction mixture as measured in terms of nitrate radicals.

The liquid reaction mixture was caused to flow through the catalyst filter and was then taken out of the reactor as a liquid substantially free of the catalyst. It thereafter flowed into a reaction liquid reservoir of a hermetically-sealed structure. It was drawn out of the reservoir once a day.

The liquid reaction mixture, which flowed out of the reactor, was sampled at intervals of two days and analyzed by gas chromatography to determine the conversion of acrylonitrile to acrylamide.

The pressure of the reaction liquid reservoir was always maintained at 4 $Kg/cm^2(G)$ by either charging or discharging nitrogen gas. In this manner, the reactor has a pressure somewhat higher than 4 $Kg/cm^2$. This pressure difference ($\Delta P$) indicates primarily the resistance of the filtration area of the catalyst filter.

$\Delta P$ was close to 0 on the first day of the reaction. It began to increase as the reaction was continued. The reaction was stopped when $\Delta P$ exceeded 2 $Kg/cm^2$.

The above reaction test was conducted by using the above-described two catalysts of different types separately. Results are given in Table 2. Namely, higher conversions and smaller $\Delta P$ were maintained during the test period of 14 days when the catalyst having the lower iron content was employed. However, with the catalyst having the higher iron content, the conversion remained low and the $\Delta P$ increased in an earlier stage of the test, resulting in termination of the test.

TABLE 2

| Run No. | 2—1 | | 2—3 | |
|---|---|---|---|---|
| Fe content (%; on Cu) | 0.24 | | 1.56 | |
| Evaluated Item | Conversion (%) | ΔP (Kg/cm) | Conversion (%) | ΔP (Kg/cm) |
| Days passed during the reaction test | | | | |
| 0 | 72 | 0 | 51 | 0 |
| 2 | 67 | 0 | 46 | 0 |
| 4 | 64 | 0 | 42 | 0.4 |
| 6 | 60 | 0 | 39 | 1.1 |
| 8 | 57 | 0.1 | Test was stopped after 7th day | |
| 10 | 54 | 0.2 | | |
| 12 | 52 | 0.2 | | |
| 14 | 50 | 0.3 | | |
| Classification | Example | | Comparative Example | |

Example 3

Using the same 5 catalysts of different lots as those employed in Example 1, reaction tests were conducted in the same manner and under the same conditions as in Example 1 except that acrylonitrile was replaced by methacrylonitrile. Results are shown in Table 3.

TABLE 3

| Run No. | Fe-content (%; based on Cu) | Conversion (%) | Classification |
|---|---|---|---|
| 3—1 | 0.10 | 54 | Example |
| 3—2 | 0.24 | 53 | ditto |
| 3—3 | 0.41 | 51 | ditto |
| 3—5 | 1.15 | 39 | Comp. Example |
| 3—6 | 1.56 | 33 | ditto |

Example 4

Using the same 2 catalysts of different lots as those employed in Example 2, continuous flow reaction tests were conducted in the same manner and under the same conditions as in Example 2 except that acrylonitrile was replaced by methacrylonitrile. Results are shown in Table 4.

TABLE 4

| Run No. | 4—1 | | 4—2 | |
|---|---|---|---|---|
| Fe content (%; on Cu) | 0.24 | | 1.56 | |
| Evaluated Item | Conversion (%) | ΔP (Kg/cm) | Conversion (%) | ΔP (Kg/cm) |
| Days passed during the reaction test | | | | |
| 0 | 61 | 0 | 45 | 0 |
| 2 | 57 | 0 | 43 | 0 |
| 4 | 55 | 0 | 42 | 0.2 |
| 6 | 53 | 0 | 40 | 0.8 |
| 8 | 50 | 0 | 38 | 1.7 |
| 10 | 47 | 0 | Test was stopped after 8th day. | |
| 12 | 45 | 0.1 | | |
| 14 | 44 | 0.1 | | |
| Classification | Example | | Comparative Example | |

Example 5

Using the same 5 catalysts of different lots as those employed in Example 1, reaction tests were conducted in the same manner and under the same conditions as in Example 1 except that acrylonitrile was replaced by nicotinonitrile. Results are shown in Table 5.

TABLE 5

| Run No. | Fe-content (%; on Cu) | Conversion (%) | Classification |
|---|---|---|---|
| 5—1 | 0.10 | 75 | Example |
| 5—2 | 0.24 | 74 | ditto |
| 5—3 | 0.41 | 71 | ditto |
| 5—4 | 1.15 | 56 | Comp. Example |
| 5—5 | 1.56 | 52 | ditto |

**Claims**

1. A process for hydrating a nitrile compound in the presence of a Raney copper catalyst in which the weight ratio of the nitrile compound to water is in the range 60:40—5:95, the reaction temperature is in the range 70—200°C and the conversion of the nitrile compound to the corresponding acid amide is in the range 10—98%; and wherein the Raney copper catalyst has an iron content in the range 0.1—0.4 wt.%

based on the copper component and the hydration reaction is carried out while maintaining the Raney copper catalyst in a suspended state, and after the reaction, catalyst and the liquid reaction mixture are separated from each other by sedimentation and/or filtration so as to recover the liquid reaction mixture.

2. A process as claimed in Claim 1, wherein the catalyst and liquid reaction mixture are separated from each other by means of a filter disposed within a reactor.

3. A process as claimed in Claim 1, wherein the catalyst and liquid reaction mixture are separated from each other by means of a thickener disposed within a reactor, and a small amount of the catalyst which is still left in a state suspended in the liquid reaction mixture is then separated by means of a filter disposed within the reactor.

4. A process as claimed in Claim 1, wherein the catalyst and liquid reaction mixture are separated from each other by means of a thickener disposed within a reactor, and a small amount of the catalyst which is still left in a state suspended in the liquid reaction mixture is then separated by means of a filter provided outside the reactor.

5. A process as claimed in any preceding claim wherein the nitrile compound is acrylonitrile.

6. A process as claimed in any preceding claim wherein the nitrile compound is methacrylonitrile.

7. A process as claimed in any preceding claim wherein the nitrile compound is nicotinonitrile.

8. A process according to any preceding claim wherein the nitrile compound to water ratio is 50:50—10:90.

**Patentansprüche**

1. Verfahren zur Hydratisierung einer Nitrilverbindung in Gegenwart eines Raney-Kupfer-Katalysators, bei dem das Gewichtsverhältnis der Nitrilverbindung zu Wasser im Bereich von 60:40 bis 5:95 liegt, die Reaktionstemperatur im Bereich von 70—200°C liegt und der Umwandlungsgrad der Nitrilverbindung zum entsprechenden Säureamid im Bereich von 10—98% liegt; und worin der Raney-Kupfer-Katalysator einen Eisengehalt im Bereich von 0,1 bis 0,4 Gew.-% bezogen auf den Kupferbestandteil aufweist und die Hydratisierungsreaktion durchgeführt wird, während der Raney-Kupfer-Katalysator in einem suspendierten Zustand gehalten wird und nach der Reaktion der Katalysator und das flüssige Reaktionsgemisch voneinander durch Sedimentierung und/oder Filtration getrennt werden, um das flüssige Reaktionsgemisch zu gewinnen.

2. Verfahren nach Anspruch 1, worin der Katalysator und das flüssige Reaktionsgemisch voneinander mittels eines Filters getrennt werden, das innerhalb eines Reaktionsgefäßes angeordnet ist.

3. Verfahren nach Anspruch 1, worin der Katalysator und das flüssige Reaktionsgemisch voneinander mittels eines Eindickers getrennt werden, der innerhalb eines Reaktors vorgesehen ist, und eine kleine Menge an Katalysator, die noch in suspendiertem Zustand im flüssigen Reaktionsgemisch zurückgeblieben ist, dann mittels eines innerhalb des Reaktors vorgesehenen Filters abgetrennt wird.

4. Verfahren nach Anspruch 1, worin der Katalysator und das flüssige Reaktionsgemisch voneinander mittels eines Eindickers getrennt werden, der innerhalb eines Reaktors vorgesehen ist, und eine kleine Menge an Katalysator, die noch im suspendierten Zustand im flüssigen Reaktionsgemisch zurückgeblieben ist, dann mittels eines außerhalb des Reaktors vorgesehenen Filters abgetrennt wird.

5. Verfahren nach einem der vorhergehenden Ansprüche, worin die Nitrilverbindung Acrylnitril ist.

6. Verfahren nach einem der vorhergehenden Ansprüche, worin die Nitrilverbindung Methacrylnitril ist.

7. Verfahren nach einem der vorhergehenden Ansprüche, worin die Nitrilverbindung Nicotinnitril ist.

8. Verfahren nach einem der vorhergehenden Ansprüche, worin das Verhältnis von Nitrilverbindung zu Wasser 50:50 bis 10:90 ist.

**Revendications**

1. Procédé pour l'hydratation d'un composé nitrile en présence d'un catalyseur de cuivre de Raney, dans lequel le rapport en poids du composé nitrile à l'eau est dans l'intervalle de 60:40—5:95, la température de réaction est dans l'intervalle de 70—200°C et la conversion du composé nitrile en l'amide d'acide correspondante est dans l'intervalle de 10—98%; et où le catalyseur de cuivre de Raney a une teneur en fer dans l'intervalle de 0,1—0,4% en poids sur la base du composant cuivre et la réaction d'hydration est réalisée tout en maintenant le catalyseur de cuivre de Raney dans un état en suspension, et après la réaction, le catalyseur et le mélange réactionnel liquide sont séparés l'un de l'autre par sédimentation et/ou filtration de telle manière à récupérer le mélange réactionnel liquide.

2. Procédé selon la revendication 1, où le catalyseur et le mélange réactionnel liquide sont séparés l'un de l'autre au moyen d'un filtre disposé à l'intérieur d'un réacteur.

3. Procédé selon la revendication 1, où le catalyseur et le mélange réactionnel liquide sont séparés l'un de l'autre au moyen d'un concentrateur disposé à l'intérieur d'un réacteur, et une petite quantité du catalyseur qui est encore laissée dans un état en suspension dans le mélange réactionnel liquide est ensuite séparée au moyen d'un filtre disposé à l'intérieur du réacteur.

4. Procédé selon la revendication 1, où le catalyseur et le mélange réactionnel liquide sont séparés l'un de l'autre au moyen d'un concentrateur disposé à l'intérieur d'un réacteur, et une petite quantité du

catalyseur qui est encore laissée dans un état en suspension dans le mélange réactionnel liquide est ensuite séparée au moyen d'un filtre disposé à l'extérieur du réacteur.

5. Procédé selon l'une des revendications précédentes, où le composé nitrile est l'acrylonitrile.

6. Procédé selon l'une quelconque des revendications précédentes, où le composé nitrile est le méthacrylonitrile.

7. Procédé selon l'une quelconque des revendications précédentes, où le composé nitrile est le nicotinonitrile.

8. Procédé selon l'une quelconque des revendications précédentes, où le rapport du composé nitrile à l'eau est de 50:50—10:90.